# EUROPEAN PATENT SPECIFICATION

(11) **EP 3 727 506 B1**
(45) Date of publication and mention of the grant of the patent: **22.04.2026**
(21) Application number: 18816104.6
(22) Date of filing: 18.12.2018
(51) Int. Cl.: A61M 5/20, A61M 5/315

(54) **APPARATUS FOR DETECTING ACTIVATION OF A DRUG DELIVERY DEVICE**
VORRICHTUNG ZUR ERKENNUNG DER AKTIVIERUNG EINER ARZNEIMITTELABGABEVORRICHTUNG
APPAREIL POUR DÉTECTER L'ACTIVATION D'UN DISPOSITIF D'ADMINISTRATION DE MÉDICAMENT

(30) Priority: 21.12.2017 EP 17306873
(43) Date of publication of application: 28.10.2020
(73) Proprietor: SANOFI, 75008 Paris (FR)
(72) Inventor: SCHABBACH, Michael, 65926 Frankfurt am Main (DE); PETRUSCHKA, Orna, 3508409 Haifa (IL)
(74) Representative: McDougall, James
(86) International application number: PCT/EP2018/085387
(87) International publication number: WO 2019/121608

(56) References cited:
- WO-A1-2014/037331
- WO-A1-2016/118736
- WO-A1-2016/128207
- WO-A1-2018/041798
- US-A1- 2007 135 756

## Description

### Field

The present disclosure relates to an apparatus for detecting activation of a drug delivery device, and in particular to an apparatus including a passive sensor for providing a wake-up signal.

### Background

A variety of diseases exists that require regular treatment by injection of a medicament. Such injection can be performed by using injection devices, which are applied either by medical personnel or by patients themselves. As an example, type-1 and type-2 diabetes can be treated by patients themselves by injection of insulin doses, for example once or several times per day. For instance, a pre-filled disposable insulin pen can be used as an injection device. Alternatively, a re-usable pen may be used. A re-usable pen allows replacement of an empty medicament cartridge by a new one. Either pen may come with a set of one-way needles that are replaced before each use. The insulin dose to be injected can then for instance be manually selected at the insulin pen by turning a dosage knob and observing the actual dose from a dose window or display of the insulin pen. The dose is then injected by inserting the needle into a suited skin portion and pressing the dosage knob or an injection button of the insulin pen. To be able to monitor insulin injection, for instance to prevent false handling of the insulin pen or to keep track of the doses already applied, it is desirable to measure information related to a condition and/or use of the injection device, such as for instance information on the injected insulin dose.

In some previous injection devices with electronic monitoring means, a wake-up switch is provided in the dose dialling button or is connected to the dose dialling button. In such devices, the electronic monitoring means are powered on when the user depresses the dose dialling button to dispense the dose. However, in such devices, the wake-up signal is received at the same moment as the dose dispensing begins, which leads to a reduction in reliability and the possibility that the dispensed dose may not be accurately recorded. On the other hand, if the electronic monitoring means are always on, so that the beginning of the dose dispensing operation is certain to be observed, then a relatively high amount of power is consumed, and the battery of the injection device may be drained before it is used. Alternatively, the battery supplied with the injection device would need to be very much larger than would otherwise be necessary.

WO 2016/118763 A1 discloses a module configured to detects the vibrations given off by dialing a click-wheel on an autoinjector and determining the selected dosage of medicine based on the dialed dosage.

US 2007/0135756 A1 discloses an injection device including an energy producing device for producing electrical energy from non-electrical energy and an energy storing device for storing the electrical energy produced.

### Summary

The invention is defined by the claims.

### Brief Description of the Figures

Example embodiments will now be described with reference to the accompanying figures, of which:
Figure 1 shows a block diagram illustrating the core components of the concept;
Figure 2 shows a perspective view of a drug delivery device containing the apparatus of Figure 1;
Figure 3 shows a block diagram of the components of the drug delivery device of Figure 2;
Figure 4a shows a supplementary device configured to be releasably attached to a drug delivery device;
Figure 4b shows a drug delivery device with the supplementary device of Figure 4a attached;
Figure 5 shows a block diagram of a system comprising the supplementary device and drug delivery device of Figure 4b according to some embodiments;
Figure 6 shows a block diagram of a system comprising the supplementary device and drug delivery device of Figure 4b according to some other embodiments;
Figure 7 shows a block diagram of a drug delivery device with a dedicated energy harvester;

### Detailed Description

In the following disclosure, embodiments will be described with reference to an insulin injection device. The present disclosure is however not limited to such application and may equally well be deployed with drug delivery devices that eject other medicaments.

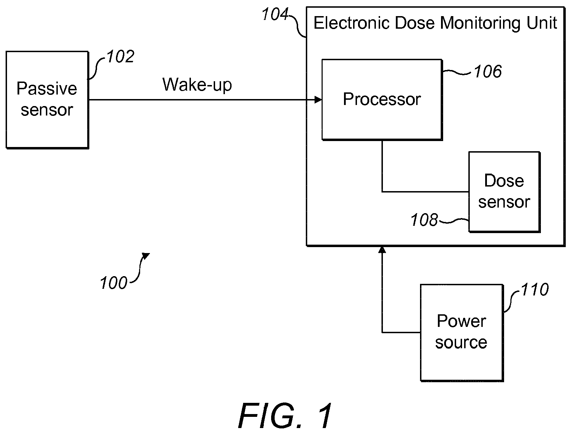

Figure 1 is a block diagram illustrating the core components of the concept. Figure 1 shows an apparatus 100 comprising a passive sensor 102, electronic dose monitoring unit 104 and power source 110. The electronic dose monitoring unit 104 comprises a processor 106 and a dose sensor 108. The electronic dose monitoring unit 104 may also be referred to as the primary electronics or the power-using electronics. The passive sensor 102 is configured to provide a wake-up signal to the electronic dose monitoring unit 104. The power source 110 is configured to provide power to the electronic dose monitoring unit 104 for powering the operation of the electronic dose monitoring unit 104.

As will be described in detail below, the apparatus 100 of Figure 1 may be embodied in a number of different ways. For example, all of the components of the apparatus 100 may be part of a drug delivery device. The components may be retained within the housing of the drug delivery device or may be housed in the dose setting dial of the drug delivery device. In some other embodiments, all of the components of the apparatus 100 are part of a supplementary device configured to be secured to a drug delivery device. For example, the supplementary device may be releasably secured to the housing or dose setting dial of the drug delivery device. In some further embodiments, some of the components of apparatus 100 are part of the drug delivery device, and some of the components are part of the supplementary device. For example, the passive sensor 102 may be part of the drug delivery device, while the electronic dose monitoring unit 104 and power source may be part of the supplementary device.

The passive sensor 102 is configured to detect vibrations of a drug delivery device. In particular, the passive sensor 102 is suitable for use with drug delivery devices which vibrate when a dose of medicament is programmed into the drug delivery device. In some embodiments, the drug delivery device is a pen type injection device. The pen injection device may have a clicker or other mechanical moving part, which causes an audible click and a vibration as each unit of medicament is programmed in. The pen injection device may have a dose dial which is manually rotated or otherwise moved by a user to set a desired dose of medicament. The primary purpose of the audible click may be to provide feedback to the user that a dose is being set and to allow the user to more easily program the desired dose. However, the vibration of the drug delivery device produced by the clicker or other mechanical moving part is detectable by the passive sensor 102, which produces an electrical signal in response. The passive sensor does not comprise any power source. The passive sensor 102 derives all of the energy for its operation from the vibration of the drug delivery device. In some embodiments, the passive sensor 102 is a piezoelectric sensor. In some other embodiments, the passive sensor 102 is an acoustic sensor. The passive sensor 102 is configured to convert the sound produced by the drug delivery device or the vibration of the drug delivery device into a wake-up signal which it provides to the electronic dose monitoring unit 104. For simplicity, in the following embodiments the passive sensor 102 will be described as sensitive to the vibration of the drug delivery device.

The electronic dose monitoring unit 104 operates in a very low power mode or an 'off' mode until it receives the wake-up signal from the passive sensor 102. In response to receiving the wake-up signal, the electronic dose monitoring unit 104, and in particular the processor 106 wakes-up so that the delivered dose of medicament can be observed and recorded. The processor 106 is configured to draw power from the power source 110 and to control the application of power to the dose sensor 108. The processor 106 may also send other control signals to the dose sensor 108. The dose sensor 108 is configured to monitor a dose delivered from the drug delivery device and to output signals to the processor 106 indicative of the amount of medicament ejected. The dose sensor 108 may be of a contact or non-contact type. For example, the dose sensor 108 may be an optical sensor, a magnetic induction sensor, a capacitive displacement sensor or a Hall Effect sensor. Alternatively, the dose sensor may comprise a number of electrodes which contact a moving part of the drug delivery device in order to measure the delivered dose.

The processor 106 may be of any suitable type such as a microprocessor, a Digital Signal Processor (DSP), Application Specific Integrated Circuit (ASIC), Field Programmable Gate Array (FPGA) or the like. The electronic dose monitoring unit 104 may also comprise one or more memories (not shown) for storing software and and/or data. For example a program memory may be provided which stores operating software which is executed by the processor 106. A dose history memory may be provided which stores data on the amount and timing of past doses.

The power source 110 may take a number of forms and will be described in greater detail in the embodiments which follow. The power source may be a battery such as a coin cell or Lithium Ion battery. Alternatively, the power source 110 may be an energy harvester and storage system. The energy harvester may collect energy from the operations of the drug delivery device, in particular from the operation of programming a dose into the drug delivery device. This energy may then be stored in a suitable way, for example by compressing or tensioning a spring or by direct conversion to electrical energy by a generator and subsequent storage in a capacitor. The stored energy is then used to power the electronic dose monitoring unit 104 during a dispensing operation, so that the dispensed dose can be observed and recorded.

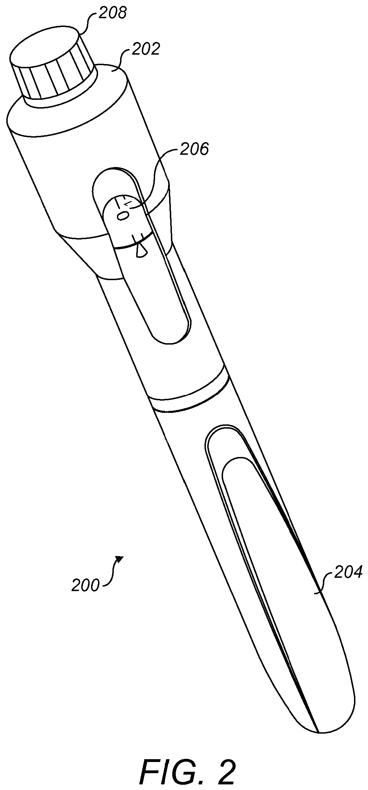

Figure 2 is a perspective view of a drug delivery device 200 containing the apparatus 100 of Figure 1. In this embodiment, the drug delivery device 200 is an injection pen for injecting a liquid medicament, such as insulin. The drug delivery device 200 comprises a housing 202 retaining the mechanical components of the dose setting and delivering mechanism and a cartridge of medicament. A needle or other medicament delivery means (not shown) is attached to a distal end of the housing 202. The drug delivery device 200 is provided with a removable cap 204 for shielding the needle. A window 206 is provided in the housing 202 to allow the currently programmed dose to be viewed by the user. A portion of the proximal end of the housing 202 may have a larger diameter in order to house the apparatus 100. Protruding from the proximal end of the housing 202 is a dose setting dial 208. In use, a user may grasp the dose setting dial 208 and rotate it to dial in a dose. In some other embodiments, the apparatus 100 may be retained in an enlarged dose setting dial or in a different area of the housing 202. In some further embodiments, the drug delivery device 200 may comprise a clip extending distally from the proximal end of the housing 202 and the apparatus may be retained inside this clip.

One advantage of using a passive sensor 102 based on vibration or acoustic detection is that the passive sensor 102 does not need to be located in a specific position or orientation relative to any other component of the drug delivery device 200. As the vibrations and/or sound travel through the body of the drug delivery device 200, the passive sensor can be located anywhere within or on the drug delivery device 200.

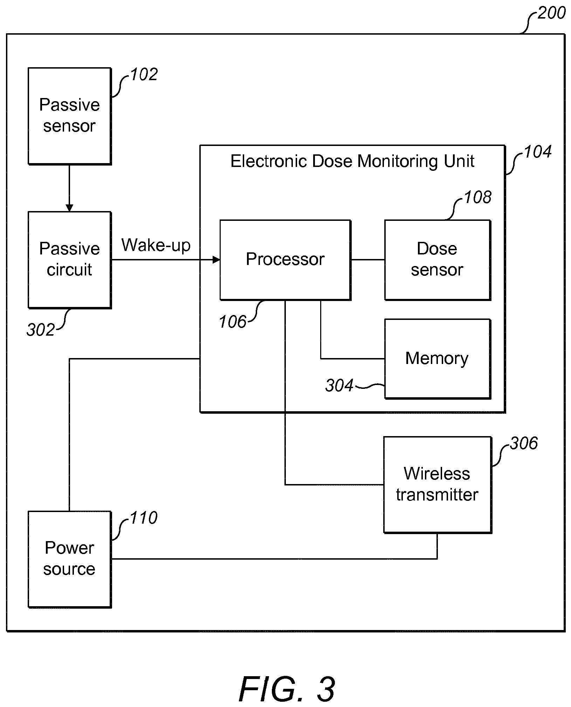

Figure 3 is a block diagram of the components of the drug delivery device 200. In addition to the components already described with relation to Figure 1 (which are not described again for conciseness), the drug delivery device 200 houses a passive circuit 302 and a wireless transmitter 306, while the electronic dose monitoring unit 104 further comprises a memory 304. The memory 304 is configured to store a dose history as measured by the dose sensor 108. The processor 106 controls the writing of information into the memory 304, which includes the amount of medicament injected and a time stamp for each medicament dispensing event. The memory may be a nonvolatile memory, such as a flash memory, so that the contents of the memory are stored even when no power is being provided to the electronic dose monitoring unit 104.

In the embodiment of Figure 3, the passive sensor 102 is configured to provide an output signal in response to vibration of the drug delivery device during dose setting to a passive circuit 302. The passive circuit 302 may comprise a passive amplifier and/or a passive filter. For example, the passive amplifier may be a passive voltage amplifier which amplifies the maximum voltage of the signal while reducing the current. The passive filter may comprise a low pass filter and/or a high pass filter which attenuates part of the signal output by the passive sensor 102. The passive circuit 302 therefore shapes the output signal so that it can be used as an input wake-up signal for the processor 106. For example, the electronic dose monitoring unit 104 may operate in a very low power mode and may be configured to wake-up upon receiving a signal of a predetermined frequency and/or minimum voltage.

The drug delivery device 200 also houses a wireless transmitter 306. The primary purpose of the wireless transmitter 306 is to transmit the dose history information stored in the memory 306. The wireless transmitter 306 may receive power directly form the power source under the control of the processor 106. In some embodiments, the wireless transmitter 306 may be a transceiver configured to exchange data with an external computing device.

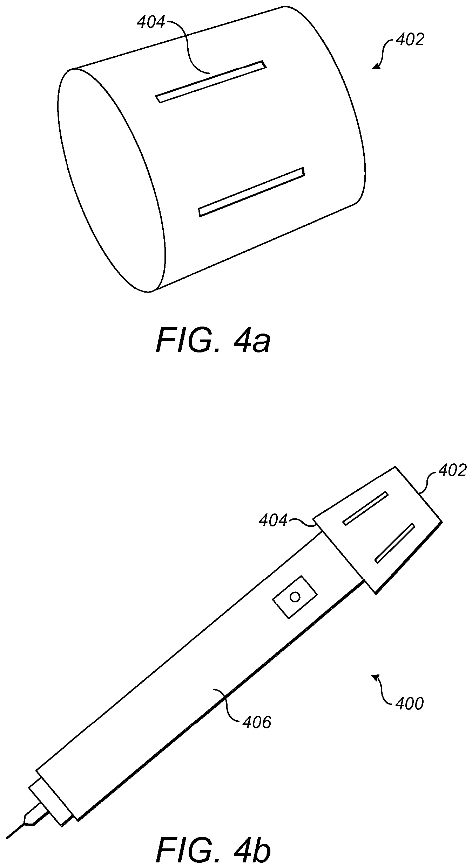

Figure 4a shows a supplementary device 402 configured to be releasably attached to a drug delivery device. The supplementary device 402 has a main body 404 which retains some or all of the components of the apparatus 100. The main body 404 of the supplementary device 402 may be shaped so as to allow it to be releasably secured to the dose setting dial 208 of the drug delivery device. For example, the main body 404 may have a cylindrical or truncated conical shape, with an open end for receiving the dose setting dial 208 and a closed end. The supplementary device 402 may therefore effectively replace the dose setting dial 208 of the drug delivery device when attached.

In some other embodiments, the supplementary device 402 has a different form and may be configured to attach to a different part of the drug delivery device, for example to the housing 202.

Figure 4b illustrates a system 400 in which the previously described concepts may be utilised. The system 400 comprises a drug delivery device 406 and the supplementary device 402 configured to be releasably attached to the drug delivery device 406. The supplementary device 402 may be that shown in Figure 4a. A user may attach and remove the supplementary device 402 from the drug delivery device 406. For example, the drug delivery device 406 may be a disposable or re-usable pen injector for injecting a number of doses of medicament. The supplementary device 402 may be compatible with a number of different models of drug delivery devices.

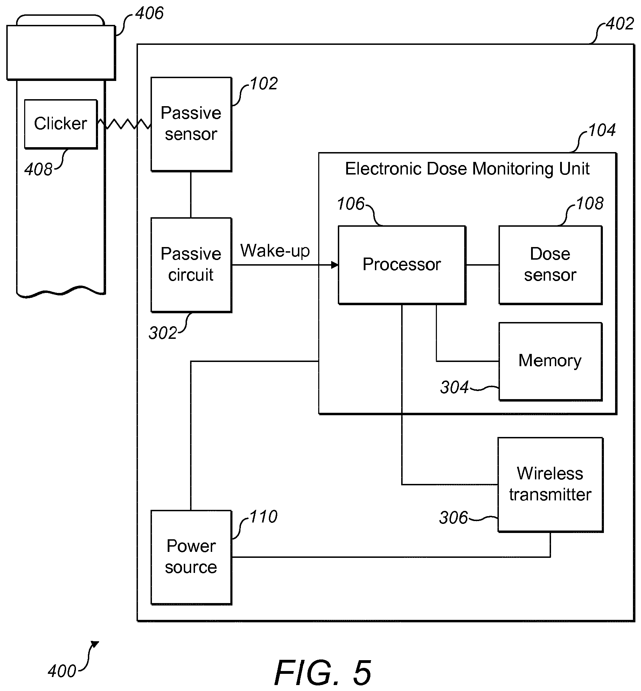

Figure 5 is a block diagram of the system 400 according to some embodiments. In the embodiment of Figure 5, the passive sensor 102, electronic dose monitoring unit 104 and power source 110 are all housed within the supplementary device 402. The supplementary device 402 is attached to the drug delivery device 406, which contains a clicker 408 or another component which causes vibration of the drug delivery device 406 when a dose of medicament is programmed into it. This vibration is transferred through to the supplementary device 402 by virtue of the attachment between the drug delivery device 406 and supplementary device 402 and is detected by the passive sensor.

The passive sensor 102 is configured to provide an output signal in response to vibration of the drug delivery device during dose setting to a passive circuit 302. The passive circuit 302 may comprise a passive amplifier and/or a passive filter. For example, the passive amplifier may be a passive voltage amplifier which amplifies the maximum voltage of the signal while reducing the current. The passive filter may comprise a low pass filter and/or a high pass filter which attenuates part of the signal output by the passive sensor 102. The passive circuit 302 therefore shapes the output signal so that it can be used as an input wake-up signal for the processor 106. For example, the electronic dose monitoring unit 104 may operate in a very low power mode and may be configured to wake-up upon receiving a signal of a predetermined frequency and/or minimum voltage.

The supplementary device 402 also houses a wireless transmitter 306. The primary purpose of the wireless transmitter 306 is to transmit the dose history information stored in the memory 304. The wireless transmitter 306 may receive power directly from the power source under the control of the processor 106. In some embodiments, the wireless transmitter 306 may be a transceiver configured to exchange data with an external computing device.

The embodiment of Figure 5 has the advantage that the drug delivery device 406 may have no electronic components, *i.e.* it is a simple mechanical device and requires no power source. The supplementary device 402 may therefore be used with existing drug delivery devices 406 of this type.

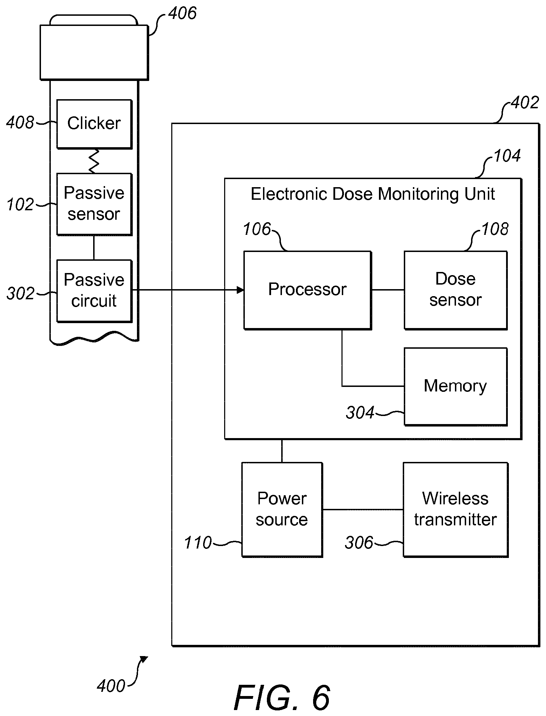

Figure 6 is a block diagram of the system 400 according to some further embodiments. In the embodiment of Figure 6, the passive sensor 102 is included in the drug delivery device 406, while the electronic dose monitoring unit 104 and power source 110 are housed within the supplementary device 402. The supplementary device 402 is attached to the drug delivery device 406, which also contains a clicker 408 or another component which causes vibration of the drug delivery device 406 when a dose of medicament is programmed into it. In this embodiment, the signal output by the passive sensor 102 is communicated from the drug delivery device 406 to the supplementary device 402, in order to wake-up the electronic dose monitoring unit 104 in the supplementary device 402. The drug delivery device 406 therefore comprises a communication interface 602 which is configured to transmit the signals to the supplementary device 402. The supplementary device 402 also comprises a communication interface 604 for receiving the signals and passing them to the electronic dose monitoring unit 104.

The interface between the drug delivery device 406 and the supplementary device 402 may be a contact interface. For example, the communication interface 602 may comprise electrical contacts on the surface of the housing of the drug delivery device 406 and the communication interface 604 may comprise corresponding electrical contacts on the surface of the supplementary device 402. The respective contacts are arranged so that when the supplementary device 402 is correctly attached to the drug delivery device 406, an electrical connection is made between the corresponding electrical contacts. Since the passive sensor 102 uses no power, in these embodiments a drug delivery device 406 can be provided which does not require any additional power source.

In some other embodiments, the interface between the drug delivery device 406 and the supplementary device 402 may be a wireless interface. The communication interface 602 of the drug delivery device 406 may be configured to use the energy harvested by the passive sensor 102 to power the sending of the signal to the supplementary device 402. Alternatively, the drug delivery device 406 may be provided with a battery for powering the wireless communication. The wireless communication may be conducted in any suitable way, for example suing a short range Bluetooth standard or NFC technology.

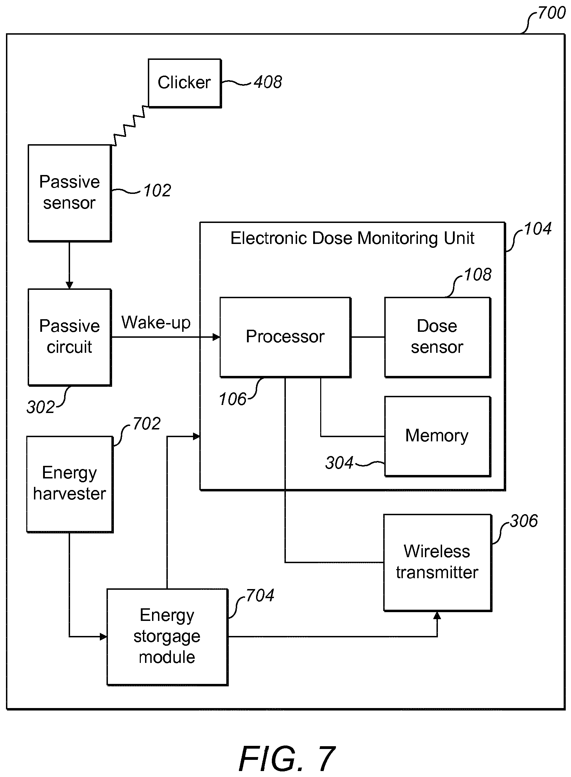

Figure 7 is a block diagram illustrating a further embodiment in which a drug delivery device 700 is provided with a dedicated energy harvester 702. The passive sensor 102 and electronic dose monitoring unit 104 (and if present the passive circuit and wireless transmitter) are all disposed within the drug delivery device 700. However, the system does not comprise a battery. The energy for powering the dose sensor 108 and processor 106 is provided from the energy harvester 702. A user may grasp the dose setting dial 208, or the supplementary device 402 where the supplementary device covers the dose setting dial and rotate to set a dose. The energy produced by this operation may be collected by the energy harvester.

The energy harvester 702 may take the form of a spring which is compressed or tensioned as a dose of medicament is programmed into the drug delivery device 406. The spring may be disposed in the main body 202 of the drug delivery device 406 or in the dose setting dial 208. When the user dispenses the medicament, the spring may be un-compressed or un-tensioned and the energy released converted into electricity for powering the electronic dose monitoring unit 104. Alternatively, the spring may be un-compressed or un-tensioned after each unit of medicament is set, and the energy stored in an energy storage module 704.

Alternatively, the energy harvester 702 may take the form of a generator. For example magnets may be disposed on an inner part of the dose setting dial 208 and coils may be retained at the proximal end of the housing 202, near the dose setting dial. When the user rotates the dose setting dial 208 a current is generated. An energy storage module 704 is provided to store the energy until needed by the electronic dose monitoring unit 104. For example a capacitor may be provided to store the charge generated. The capacitor may effectively replace the power source 110 of Figure 3 and is supplied with charge by the energy harvester 702 during dose setting. Since the passive sensor 102 uses no power, and since the power for the electronic dose monitoring unit 104 is collected by the energy harvester 702 from the dose setting operation, no additional battery is required.

Figure 7 illustrates an example of an integrated drug delivery device with an energy harvester 702 and energy storage module 704. However, systems employing an energy harvester may also be embodied as a drug delivery device and supplemental device for attachment to the drug delivery device. In one such embodiment, the clicker 408, passive sensor 102, passive circuit 302 (if present) and energy harvester 702 are part of the drug delivery device 700. The electronic dose monitoring unit 104 and wireless transmitter 306 (if present) are retained in the supplemental device. The energy storage module 704 may be part of the drug delivery device 700 or of the supplemental device, or both apparatuses may have an energy storage module. As well as transmitting the passive sensor wake-up signal from the drug delivery device 700 to the electronic dose monitoring unit 104 in the supplemental device, energy for powering the electronic dose monitoring unit 104 is also transmitted. This is preferably done through a wired connection, such as the corresponding electrical contacts discussed above.

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (anti-diabetic drugs) or 86 (oncology drugs), and Merck Index, 15th edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide.

Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir^{®}); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba^{®}); B29-N-(N-lithocholyl-gamma-glutamyl)-des(B30) human insulin; B29-N-(w-carboxyheptadecanoyl)-des(B30) human insulin and B29-N-(w-carboxyheptadecanoyl) human insulin.

Examples of GLP-1, GLP-1 analogues and GLP-1 receptor agonists are, for example, Lixisenatide (Lyxumia^{®}), Exenatide (Exendin-4, Byetta^{®}, Bydureon^{®}, a 39 amino acid peptide which is produced by the salivary glands of the Gila monster), Liraglutide (Victoza^{®}), Semaglutide, Taspoglutide, Albiglutide (Syncria^{®}), Dulaglutide (Trulicity^{®}), rExendin-4, CJC-1134-PC, PB-1023, TTP-054, Langlenatide / HM-11260C, CM-3, GLP-1 Eligen, ORMD-0901, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, TT-401, BHM-034. MOD-6030, CAM-2036, DA-15864, ARI-2651, ARI-2255, Exenatide-XTEN and Glucagon-Xten.

An example of an oligonucleotide is, for example: mipomersen sodium (Kynamro^{®}), a cholesterol-reducing antisense therapeutic for the treatment of familial hypercholesterolemia.

Examples of DPP4 inhibitors are Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc^{®}), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present disclosure include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen. Examples of antibodies are anti PCSK-9 mAb (e.g., Alirocumab), anti IL-6 mAb (e.g., Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

## Claims

1. An apparatus (100) for detecting activation of a drug delivery device (200, 406, 700), the apparatus comprising an unpowered passive sensor (102) configured to:
detect vibration of a drug delivery device caused by the programming of a dose of medicament into the drug delivery device; and
derive all of the energy for its operation from the vibration of the drug delivery device,
and wherein the apparatus is configured to use an output signal generated by the passive sensor in response to the vibration to provide a wake-up input signal to an electronic dose monitoring unit (104) for monitoring the drug delivery device.

2. An apparatus according to claim 1, further comprising a passive circuit (302) configured to transform the output signal of the passive sensor into a wake-up input signal.

3. An apparatus according to claim 2, wherein the passive circuit comprises an amplifier and a filter.

4. An apparatus according to any preceding claim, wherein the passive sensor comprises a piezoelectric sensor.

5. An apparatus according to any preceding claim, wherein the apparatus is retained in a supplementary device (402) configured to be releasably secured to the drug delivery device (406).

6. An apparatus according to any of claims 1 to 4, wherein the apparatus is integral with the drug delivery device (200, 700).

7. A drug delivery device (200, 406, 700) comprising a housing (202) retaining the apparatus of any of claims 1 to 4 and the electronic dose monitoring unit (104).

8. A system (400) comprising:
the apparatus (100) of any preceding claim; and
the electronic dose monitoring unit (104), wherein the electronic dose monitoring unit comprises a processor (106) and at least one dose sensor (108) configured to monitor a dose dispensing operation of the drug delivery device (200, 406, 700) and to determine an amount of medicament ejected from the drug delivery device.

9. A system according to claim 8, the system further comprising a wireless transmission unit (306) configured to transmit data relating to the amount of medicament ejected from the drug delivery device.

10. A system according to claim 8 or claim 9, wherein the electronic dose monitoring unit is retained in a supplementary device (402) configured to be releasably secured to the drug delivery device (406).

11. A system according to any of claims 8 to 10, the system further comprising an energy harvester (702) configured to provide power to the electronic dose monitoring unit.

12. A system according to claim 11, wherein the energy harvester is arranged to harvest energy from the programming of the dose of medicament into the drug delivery device.

13. A system according to claim 12, wherein the energy harvester is a spring configured to be compressed or tensioned by the programming of the dose of medicament into the drug delivery device and to be un-compressed or un-tensioned when the dose of medicament is ejected from the drug delivery device.

14. A system according to any of claims 11 to 13, wherein the energy harvester comprises a generator configured to produce a current in response to the programming of the dose of medicament into the drug delivery device or when the dose of medicament is ejected from the drug delivery device.

15. A system according to claim 14, wherein the system further comprises a capacitor (704) configured to store electrical energy produced by the generator.

## Patentansprüche

1. Einrichtung (100) zum Detektieren einer Aktivierung einer Medikamenten-Verabreichungsvorrichtung (200, 406, 700), wobei die Einrichtung einen unbestromten passiven Sensor (102) umfasst, der zu Folgendem ausgelegt ist:
Detektieren einer Vibration einer Medikamenten-Verabreichungsvorrichtung, die durch das Programmieren einer Medikamentendosis in der Medikamenten-Verabreichungsvorrichtung verursacht wird; und
Ableiten der gesamten Energie für ihren Betrieb aus der Vibration der Medikamenten-Verabreichungsvorrichtung,
und wobei die Einrichtung dazu ausgelegt ist, ein von dem passiven Sensor als Reaktion auf die Vibration erzeugtes Ausgangssignal zu verwenden, um ein Weckeingangssignal an eine elektronische Dosisüberwachungseinheit (104) zum Überwachen der Medikamenten-Verabreichungsvorrichtung bereitzustellen.

2. Einrichtung nach Anspruch 1, die ferner eine passive Schaltung (302) umfasst, die dazu ausgelegt ist, das Ausgangssignal des passiven Sensors in ein Weckeingangssignal umzuwandeln.

3. Einrichtung nach Anspruch 2, wobei die passive Schaltung einen Verstärker und ein Filter umfasst.

4. Einrichtung nach einem der vorhergehenden Ansprüche, wobei der passive Sensor einen piezoelektrischen Sensor umfasst.

5. Einrichtung nach einem der vorhergehenden Ansprüche, wobei die Einrichtung in einer Zusatzvorrichtung (402) gehalten ist, die dazu ausgelegt ist, lösbar an der Medikamenten-Verabreichungsvorrichtung (406) befestigt zu sein.

6. Einrichtung nach einem der Ansprüche 1 bis 4, wobei die Einrichtung mit der Medikamenten-Verabreichungsvorrichtung (200, 700) integral ist.

7. Medikamenten-Verabreichungsvorrichtung (200, 406, 700), die ein Gehäuse (202) umfasst, das die Einrichtung nach einem der Ansprüche 1 bis 4 und die elektronische Dosisüberwachungseinheit (104) hält.

8. System (400), umfassend:
die Einrichtung (100) nach einem der vorhergehenden Ansprüche; und
die elektronische Dosisüberwachungseinheit (104), wobei die elektronische Dosisüberwachungseinheit einen Prozessor (106) und mindestens einen Dosissensor (108) umfasst, der dazu ausgelegt ist, einen Dosisabgabevorgang der Medikamenten-Verabreichungsvorrichtung (200, 406, 700) zu überwachen und eine Menge an aus der Medikamenten-Verabreichungsvorrichtung ausgestoßenem Medikament zu bestimmen.

9. System nach Anspruch 8, wobei das System ferner eine drahtlose Übertragungseinheit (306) umfasst, die dazu ausgelegt ist, Daten in Bezug auf die Menge an aus der Medikamenten-Verabreichungsvorrichtung ausgestoßenem Medikament zu übertragen.

10. System nach Anspruch 8 oder Anspruch 9, wobei die elektronische Dosisüberwachungseinheit in einer Zusatzvorrichtung (402) gehalten ist, die dazu ausgelegt ist, lösbar an der Medikamenten-Verabreichungsvorrichtung (406) befestigt zu sein.

11. System nach einem der Ansprüche 8 bis 10, wobei das System ferner eine Energiegewinnungsvorrichtung (702) umfasst, die dazu ausgelegt ist, der elektronischen Dosisüberwachungseinheit Leistung bereitzustellen.

12. System nach Anspruch 11, wobei die Energiegewinnungsvorrichtung dazu eingerichtet ist, Energie aus dem Programmieren der Medikamentendosis in der Medikamenten-Verabreichungsvorrichtung zu gewinnen.

13. System nach Anspruch 12, wobei die Energiegewinnungsvorrichtung eine Feder ist, die dazu ausgelegt ist, durch das Programmieren der Medikamentendosis in der Medikamenten-Verabreichungsvorrichtung komprimiert oder gespannt zu werden und dekomprimiert oder entspannt zu werden, wenn die Medikamentendosis aus der Medikamenten-Verabreichungsvorrichtung ausgestoßen wird.

14. System nach einem der Ansprüche 11 bis 13, wobei die Energiegewinnungsvorrichtung einen Generator umfasst, der dazu ausgelegt ist, als Reaktion auf das Programmieren der Medikamentendosis in der Medikamenten-Verabreichungsvorrichtung oder wenn die Medikamentendosis aus der Medikamenten-Verabreichungsvorrichtung ausgestoßen wird, einen Strom zu erzeugen.

15. System nach Anspruch 14, wobei das System ferner einen Kondensator (704) umfasst, der dazu ausgelegt ist, von dem Generator erzeugte elektrische Energie zu speichern.

## Revendications

1. Appareil (100) destiné à détecter l'activation d'un dispositif d'administration de médicament (200, 406, 700), l'appareil comprenant un capteur passif non alimenté (102) configuré pour :
détecter la vibration d'un dispositif d'administration de médicament provoquée par le réglage d'une dose de substance médicamenteuse dans le dispositif d'administration de médicament ; et
obtenir toute l'énergie nécessaire à son fonctionnement à partir de la vibration du dispositif d'administration de médicament,
et l'appareil étant configuré pour utiliser un signal de sortie généré par le capteur passif en réponse à la vibration afin de fournir un signal d'entrée de réveil à une unité électronique de surveillance de dose (104) destinée à surveiller le dispositif d'administration de médicament.

2. Appareil selon la revendication 1, comprenant en outre un circuit passif (302) configuré pour transformer le signal de sortie du capteur passif en un signal d'entrée de réveil.

3. Appareil selon la revendication 2, dans lequel le circuit passif comprend un amplificateur et un filtre.

4. Appareil selon l'une quelconque des revendications précédentes, dans lequel le capteur passif comprend un capteur piézoélectrique.

5. Appareil selon l'une quelconque des revendications précédentes, l'appareil étant retenu dans un dispositif supplémentaire (402) configuré pour être fixé de manière amovible au dispositif d'administration de médicament (406).

6. Appareil selon l'une quelconque des revendications 1 à 4, l'appareil étant solidaire du dispositif d'administration de médicament (200, 700).

7. Dispositif d'administration de médicament (200, 406, 700) comprenant un boîtier (202) retenant l'appareil selon l'une quelconque des revendications 1 à 4 et l'unité électronique de surveillance de dose (104).

8. Système (400) comprenant :
l'appareil (100) selon l'une quelconque des revendications précédentes ; et
l'unité électronique de surveillance de dose (104),
l'unité électronique de surveillance de dose comprenant un processeur (106) et au moins un capteur de dose (108) configuré pour surveiller une opération de distribution de dose du dispositif d'administration de médicament (200, 406, 700) et pour déterminer une quantité de substance médicamenteuse éjectée du dispositif d'administration de médicament.

9. Système selon la revendication 8, le système comprenant en outre une unité de transmission sans fil (306) configurée pour transmettre des données relatives à la quantité de substance médicamenteuse éjectée du dispositif d'administration de médicament.

10. Système selon la revendication 8 ou la revendication 9, dans lequel l'unité électronique de surveillance de dose est retenue dans un dispositif supplémentaire (402) configuré pour être fixé de manière amovible au dispositif d'administration de médicament (406).

11. Système selon l'une quelconque des revendications 8 à 10, le système comprenant en outre un récupérateur d'énergie (702) configuré pour alimenter l'unité électronique de surveillance de dose.

12. Système selon la revendication 11, dans lequel le récupérateur d'énergie est agencé pour récupérer de l'énergie à partir du réglage de la dose de substance médicamenteuse dans le dispositif d'administration de médicament.

13. Système selon la revendication 12, dans lequel le récupérateur d'énergie est un ressort configuré pour être comprimé ou tendu par le réglage de la dose de substance médicamenteuse dans le dispositif d'administration de médicament et pour être décomprimé ou détendu lorsque la dose de substance médicamenteuse est éjectée du dispositif d'administration de médicament.

14. Système selon l'une quelconque des revendications 11 à 13, dans lequel le récupérateur d'énergie comprend un générateur configuré pour produire un courant en réponse au réglage de la dose de substance médicamenteuse dans le dispositif d'administration de médicament ou lorsque la dose de substance médicamenteuse est éjectée du dispositif d'administration de médicament.

15. Système selon la revendication 14, le système comprenant en outre un condensateur (704) configuré pour stocker l'énergie électrique produite par le générateur.
